# EUROPEAN PATENT APPLICATION

(11) **EP 4 349 991 A1**
(43) Date of publication of application: **10.04.2024**
(21) Application number: 22833363.9
(22) Date of filing: 31.03.2022
(51) Int. Cl.: C12N 15/77, C12N 9/02, C12P 13/14

(54) **STRAIN FOR PRODUCING HIGHLY CONCENTRATED L-GLUTAMIC ACID, AND L-GLUTAMIC ACID PRODUCTION METHOD USING SAME**

(30) Priority: 30.06.2021 KR 20210085738
(71) Applicant: CJ Cheiljedang Corporation, Seoul 04560 (KR)
(72) Inventor: KWON, Nara, Seoul 04560 (KR); BONG, Hyun-Ju, Seoul 04560 (KR); LEE, Jin Nam, Seoul 04560 (KR); LEE, Ah Reum, Seoul 04560 (KR); HEO, Jung Ok, Seoul 04560 (KR)
(74) Representative: Regimbeau
(86) International application number: PCT/KR2022/004562
(87) International publication number: WO 2023/277307

(57) **Abstract**

The present application relates to a strain for producing L-glutamic acid in high concentration and a method for producing L-glutamic acid using the same.

## Description

### [Technical Field]

The present application relates to a strain for producing L-glutamic acid in high concentration and a method for producing L-glutamic acid using the same.

### [Background Art]

L-Glutamic acid is a representative amino acid produced by fermentation and has a unique, distinctive taste, and thus is an important amino acid widely used in the field of food industry as well as in the medical field and other animal feed fields. It is known that methods for producing L-glutamic acid include a method using a microorganism of the genus *Corynebacterium, Escherichia coli,* or microorganisms of the genera *Bacillus, Streptomyces, Penicillium, Klebsiella, Erwinia, Pantoea, etc.* (U.S. Patent Nos. 3,220,929 and 6,682,912).

Currently, various studies are being conducted for the development of microorganisms and fermentation process technology that produce L-glutamic acid with high efficiency. For example, in a microorganism of the genus *Corynebacterium,* a target material-specific approach such as increasing the expression of a gene encoding an enzyme involved in amino acid biosynthesis or removing a gene unnecessary for amino acid biosynthesis is mainly used for enhancement of L-glutamic acid production yield.

### [Disclosure]

### [Technical Problem]

The present inventors have made extensive efforts to produce L-glutamic acid in high yield, and as a result, they have confirmed that the L-glutamic acid producing ability was increased by the inactivated VKOR protein, thereby completing the present invention.

### [Technical Solution]

An object of the present application is to provide a microorganism of the genus *Corynebacterium,* in which VKOR protein (vitamin K epoxide reductase family protein) is inactivated.

Another object of the present application is to provide a method for producing L-glutamic acid, including: culturing a microorganism of the genus *Corynebacterium,* in which VKOR protein is inactivated, in a medium.

Still another object of the present application is to provide a composition for producing L-glutamic acid, including: a microorganism of the genus *Corynebacterium,* in which VKOR protein is inactivated; a medium for culturing the same; or a combination thereof.

Still another object of the present application is to provide a method for producing a microorganism of the genus *Corynebacterium,* including: inactivating VKOR protein.

Still another object of the present application is to provide a composition for producing L-glutamic acid, including: a microorganism of the genus *Corynebacterium,* in which VKOR protein is inactivated; a medium for culturing the same; or a combination thereof.

Still another object of the present application is to provide a method for producing a microorganism of the genus *Corynebacterium,* including: inactivating VKOR protein.

Still another object of the present application is to provide the use of L-glutamic acid production of a microorganism of the genus *Corynebacterium,* in which VKOR protein is inactivated.

### [Advantageous Effects]

The L-glutamic acid-producing microorganism of the genus *Corynebacterium,* in which the VKOR protein of the present application is inactivated, can produce L-glutamic acid in high yield, and thus can be effectively used for industrial production of L-glutamic acid.

### [Detailed Description of Preferred Embodiments]

The present application will be described in detail as follows. Meanwhile, each description and embodiment disclosed herein can be applied to other descriptions and embodiments, respectively. That is, all combinations of various elements disclosed herein fall within the scope of the present application. Further, the scope of the present application is not limited by the specific description described below. Additionally, those of ordinary skill in the art may be able to recognize or confirm, using only conventional experimentation, many equivalents to the particular aspects of the invention described herein. Furthermore, it is also intended that these equivalents be included in the present application.

One aspect of the present application provides a microorganism of the genus *Corynebacterium,* in which VKOR protein (vitamin K epoxide reductase family protein) is inactivated.

As used herein, the term "VKOR (vitamin K epoxide reductase family protein)" refers to an enzyme having the activity of reducing vitamin K 2,3-epoxide and vitamin K to vitamin K hydroquinone (Vitamins & Hormones Volume 78, 2008, pages 103-130).

In one example, the VKOR protein of the present application may be derived from a microorganism. The microorganism may be specifically derived from a microorganism of the genus *Corynebacterium.* More specifically, it may be derived from *Corynebacterium glutamicum, Corynebacterium deserti, Corynebacterium crudilactis, Corynebacterium efficiens, Corynebacterium callunae, etc.,* but is not limited thereto.

The amino acid sequence of the VKOR protein may be encoded by the *VKOR* gene. For example, the *VKOR* gene may be *NCgl0775* derived from *Corynebacterium glutamicum* ATCC13032 or *BBD29_04485* derived from *Corynebacterium glutamicum* ATCC13869, but is not limited thereto.

The VKOR protein of the present application may include or consists of a polypeptide represented by an amino acid sequence having a sequence homology of 80% or more with the amino acid sequence of SEQ ID NO: 1. Additionally, the VKOR protein of the present application may have an amino acid sequence having a sequence homology of 80% or more with the amino acid sequence of SEQ ID NO: 1 or may essentially consist of the amino acid sequence. Specifically, the protein may consist of a polypeptide represented by the amino acid sequence of SEQ ID NO: 1.

The amino acid sequence of SEQ ID NO: 1 can be obtained from NIH GenBank, a known database. In the present application, the amino acid sequence of SEQ ID NO: 1 may include an amino acid sequence having a homology or identity of at least 70%, 75%, 80%, 85%, 90%, 95%, 96%, 97%, 98%, 99%, 99.5%, 99.7%, or 99.9% with the amino acid sequence of SEQ ID NO: 1. Additionally, it is apparent that proteins having an amino acid sequence, in which a part of the amino acid sequence is deleted, modified, substituted, conservatively substituted or added, may fall within the scope of the present application, as long as the amino acid sequence has such homology or identity and shows an efficacy corresponding to that of the protein including the amino acid sequence of SEQ ID NO: 1.

For example, it may include sequence additions or deletions, naturally occurring mutations, silent mutations or conservative substitutions that do not alter the function of the protein of the present application at the N-terminus, C-terminus, and/or within the amino acid sequence.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. Typically, conservative substitutions may have little or no effect on the activity of a protein or polypeptide.

As used herein, the term "homology" or "identity" refers to a degree of relatedness between two given amino acid sequences or nucleotide sequences, and may be expressed as a percentage. The terms homology and identity may often be used interchangeably with each other.

The sequence homology or identity of conserved polypeptide or polynucleotide sequences may be determined by standard alignment algorithms and can be used with a default gap penalty established by the program being used. Substantially, homologous or identical sequences are generally expected to hybridize to all or part of the entire length of the sequences under moderate or high stringent conditions. It is apparent that hybridization with polynucleotides containing general codon or degenerate codons in hybridizing polynucleotides is also included.

Whether any two polynucleotide sequences have a homology, similarity or identity may be, for example, determined by a known computer algorithm such as the "FASTA" program using default parameters (Pearson et al., (1988) Proc. Natl. Acad. Sci. USA 85:2444). Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polypeptides or polynucleotides may be determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity or identity as the value obtained by dividing the number of similarly aligned symbols (*i*.*e*., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

In the present application, the polynucleotide encoding the VKOR protein may be the *VKOR* gene. In one example, the *VKOR* gene may include *NCgl0775* or *BBD29_04485* gene, *etc.,* as described above.

As used herein, the term "polynucleotide", which is a polymer of nucleotides composed of nucleotide monomers connected in a lengthy chain by a covalently bond, is a DNA or RNA strand having at least a certain length. More specifically, it may refer to a polynucleotide fragment encoding the protein.

The polynucleotide encoding the VKOR protein of the present application may include a nucleotide sequence encoding the amino acid sequence represented by SEQ ID NO: 1. As an example of the present application, the polynucleotide of the present application may have or include the sequence of SEQ ID NO: 2. In addition, the polynucleotide of the present application may consist or consist essentially of the sequence of SEQ ID NO: 2. Specifically, the VKOR protein may be encoded by the polynucleotide represented by the nucleotide sequence of SEQ ID NO: 2.

The polynucleotide of the present application may undergo various modifications in the coding region within the scope that does not change the amino acid sequence of the VKOR protein, due to codon degeneracy or in consideration of the codons preferred in an organism in which the VKOR protein of the present application is to be expressed. Specifically, the polynucleotide of the present application may have or include a nucleotide sequence having a homology or identity of 70% or more, 75% or more, 76% or more, 85% or more, 90% or more, 95% or more, 96% or more, 97% or more, or 98% or more, with the sequence of SEQ ID NO: 2, or may consist or consist essentially of a nucleotide sequence having a homology or identity of 70% or more, 75% or more, 76% or more, 85% or more, 90% or more, 95% or more, 96% or more, 97% or more, or 98% or more, with the sequence of SEQ ID NO: 2, but is not limited thereto.

Additionally, the polynucleotide of the present application may include a probe that may be prepared from a known gene sequence, for example, any sequence which can hybridize with a sequence complementary to all or part of the polynucleotide sequence of the present application under stringent without limitation. The "stringent conditions" refers to conditions under which specific hybridization between polynucleotides is allowed. Such conditions are specifically described in the literature (J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York, 9.50-9.51, 11.7-11.8). For example, the stringent conditions may include conditions under which polynucleotides having a high homology or identity of 70% or more, 75% or more, 76% or more, 85% or more, 90% or more, 95% or more, 96% or more, 97% or more, 98% or more, or 99% or more are hybridized with each other and polynucleotides having a homology or identity lower than the above homologies or identities are not hybridized with each other, or washing conditions of Southern hybridization, that is, washing once, specifically twice or three times at a salt concentration and a temperature corresponding to 60°C, 1× SSC, 0.1% SDS, specifically 60°C, 0.1× SSC, 0.1% SDS, and more specifically 68°C, 0.1× SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present application may include isolated nucleotide fragments complementary to the entire sequence as well as nucleic acid sequences substantially similar thereto.

Specifically, polynucleotides having a homology or identity with the polynucleotide of the present application may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e*.*g*., Sambrook *et al.*)*.*

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, and may be a microorganism including genetic modification to produce a desired polypeptide, protein or product.

As used herein, the term "inactivation" of a polypeptide activity is a comprehensive concept including both reduced or no activity compared to its endogenous activity. The inactivation may be used interchangeably with terms such as weakening, deficiency, down-regulation, decrease, reduce, attenuation, *etc.*

The inactivation may also include a case where the polypeptide activity itself is decreased or removed compared to the activity of the polypeptide originally possessed by a microorganism due to a mutation of the polynucleotide encoding the polypeptide; a case where the overall level of intracellular polypeptide activity and/or concentration (expression level) is decreased compared to a natural strain due to the inhibition of expression of the gene of the polynucleotide encoding the polypeptide, or the inhibition of translation into the polypeptide, *etc*.; a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression "the polypeptide activity is "inactivated, weakened, deficient, decreased, down-regulated, reduced or attenuated" compared to its endogenous activity" means that the polypeptide activity is decreased compared to the activity of a particular polypeptide originally possessed by a parent strain before transformation or a non-modified microorganism.

The inactivation of the polypeptide activity can be performed by any method known in the art, but the method is not limited thereto, and can be achieved by applying various methods well known in the art (*e*.*g*., Nakashima N et al., Bacterial cellular engineering by genome editing and gene silencing. Int J Mol Sci. 2014;15(2):2773-2793, Sambrook et al. Molecular Cloning 2012, *etc.*)*.*

Specifically, the inactivation of the polypeptide activity of the present application may be achieved by:
1) deleting a part or all of the gene encoding the polypeptide;
2) modifying the expression regulatory region (expression regulatory sequence) such that the expression of the gene encoding the polypeptide is decreased;
3) modifying the amino acid sequence constituting the polypeptide such that the polypeptide activity is removed or weakened (*e*.*g*., deletion/substitution/addition of one or more amino acids on the amino acid sequence);
4) modifying the gene sequence encoding the polypeptide such that the polypeptide activity is removed or weakened (*e*.*g*., deletion/substitution/addition of one or more of nucleotides on the nucleotide sequence of the polypeptide gene to encode a polypeptide that has been modified to remove or weaken the activity of the polypeptide;
5) modifying the nucleotide sequence encoding the initiation codon, Shine-Dalgarno sequence or 5'-UTR of the gene transcript encoding the polypeptide;
6) introducing an antisense oligonucleotide (*e*.*g*., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide; or
9) a combination of two or more selected from the methods 1) to 8) above, but is not particularly limited thereto.

### For example:

The 1) method of deleting a part or all of the gene encoding the polypeptide may be achieved by deleting all of the polynucleotide encoding the endogenous target polypeptide within the chromosome, or by replacing the polynucleotide with a polynucleotide or a marker gene having a partially deleted nucleic acids.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome binding site, and a sequence for regulating transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of a gene may be inhibited or weakened by introducing a mutation into the polynucleotide sequence to form a stop codon, but is not limited thereto.

The 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (*e*.*g*., antisense RNA), which binds complementary to the gene transcript encoding the polypeptide can be found in the literature (Weintraub, H. et al., Antisense-RNA as a molecular tool for genetic analysis, Reviews - Trends in Genetics, Vol. 1(1) 1986).

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of the gene sequence encoding the polypeptide may be achieved by forming an antisense nucleotide complementary to the gene transcript encoding the polypeptide to weaken the activity.

As used herein, the term "enhancement" of a polypeptide activity means that the activity of a polypeptide is increased compared to its endogenous activity. The enhancement may be used interchangeably with terms such as activation, up-regulation, overexpression, increase, *etc.* In particular, the terms activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide can be confirmed by the increase in the level of activity of the polypeptide, expression level, or the amount of product excreted from the polypeptide.

The enhancement of the activity of the polypeptide can be applied by various methods well known in the art, and is not limited as long as it can enhance the activity of the target polypeptide compared to that of the microorganism before modification. Specifically, genetic engineering and/or protein engineering well known to those skilled in the art, which is a common method of molecular biology, may be used, but the method is not limited thereto (*e*.*g*., Sitnicka et al. Functional Analysis of Genes. Advances in Cell Biology. 2010, Vol. 2. 1-16, Sambrook et al. Molecular Cloning 2012, *etc.*)*.*

Specifically, the enhancement of the polypeptide of the present application may be achieved by:
1) increasing the intracellular copy number of a polynucleotide encoding the polypeptide;
2) replacing the expression regulatory region of a gene encoding the polypeptide on the chromosome with a sequence having a strong activity;
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e*.*g*., modifying the polynucleotide sequence of the polypeptide gene to encode a polypeptide that has been modified to enhance the activity of the polypeptide);
6) introducing a foreign polynucleotide having the activity of the polypeptide or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

### More specifically,

The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto.

The 2) method of replacing the expression regulatory region (or expression regulatory sequence) of a gene encoding the polypeptide on the chromosome with a sequence having a strong activity may be achieved by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome binding site, and a sequence regulating the termination of transcription and translation. In one example, the method may include replacing the original promoter with a strong promoter, but is not limited thereto.

Examples of the strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13(sm3) promoter (US 10584338 B2), O2 promoter (US 10273491 B2), tkt promoter, yccA promoter, *etc.,* but are not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or polynucleotide sequence modified to have a stronger activity, or an amino acid sequence or polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is the same as described above.

The 6) method of introducing a foreign polynucleotide having the activity of the polypeptide may be achieved by introducing into a host cell a foreign polynucleotide encoding a polypeptide that exhibits the same or similar activity to the polypeptide. The foreign polynucleotide may be used without limitation regardless of its origin or sequence as long as it exhibits the same or similar activity to the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

In addition, the 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration (expression level) of the polypeptide is increased relative to the activity or concentration of the polypeptide expressed in a wild-type or a microorganism before modification, or that the amount of product produced from the polypeptide is increased, but is not limited thereto.

The modification of a part or all of the polynucleotide in the microorganism of the present application may be achieved by (a) homologous recombination using a vector for chromosomal insertion in the microorganism or genome editing using engineered nuclease (*e*.*g*., CRISPR-Cas9) and/or (b) may be induced by light, such as ultraviolet rays and radiation, *etc.,* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into a microorganism to induce homologous recombination. The injected nucleotide sequence or the vector may include a dominant selection marker, but is not limited thereto.

The vector of the present application may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function independently from the host genome, or may integrate into genome thereof.

The vector used in the present application is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDC, pBR, pUC, pBluescriptll, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface proteins, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or a microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome binding site, or a translation terminator. The expression cassette may be in the form of a self-replicable expression vector. Additionally, the polynucleotide may be introduced into the host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target polypeptide of the present application.

The microorganism of the present application may be a microorganism in which VKOR protein or a polynucleotide encoding the protein is inactivated; or a microorganism (*i*.*e*., a recombinant microorganism) which is genetically modified through a vector such that VKOR protein or a polynucleotide encoding the protein is inactivated, but is not limited thereto. The vector is as described above.

The microorganism of the present application may be a microorganism having the ability to produce L-glutamic acid.

The microorganism of the present application may be a microorganism having the ability to naturally produce L-glutamic acid, or a microorganism in which the ability to produce L-glutamic acid has been imparted by inactivating the VKOR protein or the polynucleotide encoding the protein into a parent strain having no ability to produce L-glutamic acid, but is not limited thereto.

In one example, the recombinant microorganism of the present application is a microorganism which is transformed with a vector such that the VKOR protein or the polynucleotide encoding the protein is inactivated, and thus the VKOR protein or the polynucleotide encoding the protein is inactivated, and may include all microorganisms capable of producing L-glutamic acid by inactivating the VKOR protein or the polynucleotide encoding the protein.

For the purpose of the present application, the recombinant microorganism of the present application may be a microorganism in which the L-glutamic acid producing ability has been increased by inactivation of the VKOR protein or the polynucleotide encoding the same in a natural wild-type microorganism or a microorganism for producing L-glutamic acid by including the VKOR protein or the polynucleotide encoding the protein, as compared to the natural wild-type microorganism or microorganism for producing L-glutamic acid by including the VKOR protein or the polynucleotide encoding the protein, but is not limited thereto. In one example, the non-modified microorganism, in which the VKOR protein is not inactivated, which is a target strain to be compared to determine the increase in the L-glutamic acid producing ability, may be a *Corynebacterium glutamicum* ATCC13869 strain, in which the *odhA* gene known as an L-glutamic acid-producing strain is deleted, or a *Corynebacterium glutamicum* BL2 strain known as an L-glutamic acid producing NTG mutant strain (KFCC11074, Korean Patent No. 10-0292299), but is not limited thereto.

In one example, the recombinant strain having an increased producing ability may have an increased L-glutamic acid producing ability by about 1% or more, specifically about 1% or more, about 2.5% or more, about 5% or more, about 6% or more, about 7% or more, about 8% or more, about 9% or more, about 10% or more, about 14.3% or more, about 20% or more, about 28.6% or more, about 30% or more, about 31.9% or more, about 33.3% or more, about 37.7% or more, about 40% or more, about 42.9% or more, about 46.3% or more, about 50% or more or about 52.4% or more (the upper limit is not particularly limited, for example, about 200% or less, about 150% or less, about 100% or less, about 50% or less, about 40% or less, about 30% or less, about 20% or less or about 15% or less), as compared to that of the parent strain before modification or a non-modified microorganism, but is not limited thereto, as long as it has an increased + value compared to the production ability of the parent strain before modification or non-modified microorganism. In another example, the microorganism having an increased production ability may have an increased L-glutamic acid producing ability by about 1.01 times or more, about 1.02 times or more, about 1.03 times or more, about 1.05 times or more, about 1.06 times or more, about 1.07 times or more, about 1.08 times or more, about 1.09 times or more, about 1.1 times or more, about 1.14 times or more, about 1.28 times or more, about 1.32 times or more, about 1.33 times or more, about 1.37 times or more, about 1.43 times or more, about 1.46 times or more or about 1.52 times or more (the upper limit is not particularly limited, for example, about 10 times or less, about 5 times or less, about 3 times or less, or about 2 times or less), as compared to that of the parent strain before modification or non-modified microorganism, but is not limited thereto.

As used herein, the term "non-modified microorganism" does not exclude a strain containing a mutation that may occur naturally in a microorganism, and may refer to a wild-type strain or a natural-type strain itself, or a strain before the trait is altered due to genetic modification caused by natural or artificial factors. For example, the non-modified microorganism may refer to a strain in which the VKOR protein described in the present specification or the polynucleotide encoding the protein is not inactivated or a strain before inactivation thereof. The "non-modified microorganism" may be used interchangeably with "strain before modification", "microorganism before modification", "non-mutant strain", "non-modified strain", "non-mutant microorganism" or "reference microorganism".

In one embodiment, the microorganism of the present application may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris* or *Corynebacterium flavescens,* and specifically *Corynebacterium glutamicum,* but is not limited thereto.

In another example, the recombinant microorganism of the present application may be a microorganism in which the activity of a part of the protein in the L-glutamic acid biosynthesis pathway is additionally enhanced, or the activity of a part of the protein in the L-glutamic acid degradation pathway is additionally inactivated, thereby enhancing the L-glutamic acid producing ability.

Specifically, the microorganism of the present application may be a microorganism in which the OdhA protein is further inactivated or the *odhA* gene is further deleted. More specifically, the microorganism of the present application may be a *Corynebacterium glutamicum* in which the OdhA protein is inactivated in *Corynebacterium glutamicum* ATCC13869, or a microorganism in which the *odhA* gene is deleted from *Corynebacterium glutamicum* ATCC13869. The OdhA protein may include the amino acid sequence (SEQ ID NO: 32) of NCBI Sequence ID WP_060564343.1, and the *odhA* gene may include the nucleotide sequence of NCBI GenBank BBD29_06050, but the present application is not limited thereto.

However, the OdhA protein inactivation or *odhA* gene deletion is only an example, and the present application is not limited thereto. Additionally, the microorganism of the present application may be a microorganism in which the protein activity of various known L-glutamic acid biosynthesis pathways is enhanced or the protein activity of the degradation pathway is inactivated.

Another aspect of the present application provides a method for producing L-glutamic acid, including: culturing a microorganism of the genus *Corynebacterium,* in which VKOR protein is inactivated, in a medium.

The method for producing L-glutamic acid of the present application may include a step of culturing a microorganism in which the VKOR protein or a polynucleotide encoding the protein in inactivated; or a microorganism of the genus *Corynebacterium,* which is genetically modified through a vector such that the VKOR protein or a polynucleotide encoding the protein in inactivated, in a medium.

As used herein, the term "cultivation" means that the microorganism of the present application is grown under appropriately controlled environmental conditions. The cultivation process of the present application may be performed in a suitable culture medium and culture conditions known in the art. Such a cultivation process may be easily adjusted for use by those skilled in the art according to the microorganism to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the present application as a main ingredient, and it supplies nutrient materials and growth factors, along with water that is essential for survival and growth. Specifically, the medium and other culture conditions used for culturing the microorganism of the present application may be any medium used for conventional cultivation of microorganisms without any particular limitation. However, the microorganism of the present application may be cultured under aerobic conditions in a conventional medium containing an appropriate carbon source, nitrogen source, phosphorus source, inorganic compound, amino acid, and/or vitamin, while adjusting temperature, pH, *etc.*

Specifically, the culture medium for the microorganism of the genus *Corynebacterium* can be found in the literature ("Manual of Methods for General Bacteriology" by the American Society for Bacteriology (Washington D.C., USA, 1981)).

In the present application, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, corn steep liquor, *etc.* Specifically, carbohydrates such as glucose and sterilized pretreated molasses (i.e., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compound may include sodium chloride, calcium chloride, iron chloride, magnesium sulfate, iron sulfate, manganese sulfate, calcium carbonate, *etc.* Additionally, amino acids, vitamins, and/or appropriate precursors, *etc.* may be included. These constituting ingredients or precursors may be added to a medium in a batch or continuous manner, but these phosphorus sources are not limited thereto.

The pH of a medium may be adjusted during the cultivation of the microorganism of the present application by adding a compound such as ammonium hydroxide, potassium hydroxide, ammonia, phosphoric acid, sulfuric acid, *etc.* to the medium in an appropriate manner. Additionally, during the cultivation, an antifoaming agent such as fatty acid polyglycol ester may be added to prevent foam generation. In addition, oxygen or oxygen-containing gas may be injected into the medium in order to maintain an aerobic state of the medium; or nitrogen, hydrogen, or carbon dioxide gas may be injected without the injection of gas in order to maintain an anaerobic or microaerobic state of the medium, but the gas is not limited thereto.

The medium temperature in the cultivation of the present application may be in a range from 20°C to 45°C, and specifically from 25°C to 40°C, and the cultivation may be carried out for about 10 to 160 hours, but is not limited thereto.

The L-glutamic acid produced by the cultivation of the present application may be released into the medium or remain in the cells.

The method for producing L-glutamic acid of the present application may further include a step of preparing the microorganism of the present application, a step of preparing a medium for culturing the microorganism, or a combination thereof (regardless of the order, in any order), for example, prior to the culturing step.

The method for producing L-glutamic acid of the present application may further include a step of recovering L-glutamic acid from the culture medium (medium on which the culture was grown) or the cultured microorganism. The recovering step may be further included after the culturing step.

In the recovering step, desired L-glutamic acids may be collected using the method of culturing a microorganism of the present application, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC or a combination thereof may be used, and the desired L-glutamic acids can be recovered from the medium or microorganisms using suitable methods known in the art.

Further, the method for producing L-glutamic acid of the present application may further include a purification process, which may be performed using an appropriate method known in the art. In one example, when the method for producing L-glutamic acid of the present application includes both a recovering step and a purification step, the recovering step and the purification step may be performed continuously or intermittently regardless of the order, or simultaneously or may be integrated into one step, but the method is not limited thereto.

In the method of the present application, the VKOR protein, polynucleotides, vectors and microorganism, *etc.* are the same as described in other aspects.

Still another aspect of the present application provides a composition for producing L-glutamic acid, including: a microorganism of the genus *Corynebacterium,* in which VKOR protein is inactivated; a medium for culturing the same; or a combination thereof.

The composition of the present application may further include any suitable excipient commonly used in the composition for producing L-glutamic acid, and such excipient may be, for example, a preservative, a wetting agent, a dispersing agent, a suspending agent, a buffering agent, a stabilizing agent, or an isotonic agent, *etc.,* but is not limited thereto.

Yet another aspect of the present application provides a method for producing a microorganism of the genus *Corynebacterium,* including inactivating VKOR protein.

Even another aspect of the present application provides the use of L-glutamic acid production of a microorganism of the genus *Corynebacterium,* in which VKOR protein is inactivated.

The VKOR protein, inactivation, microorganism of the genus *Corynebacterium, etc.,* are the same as described in other aspects.

### [Mode for Carrying Out the Invention]

Hereinafter, the present application will be described in detail by way of Examples. However, these Examples are merely preferred Examples given for illustrative purposes, and thus, the scope of the present application is not intended to be limited to or by these Examples. Meanwhile, technical features which are not described herein can be sufficiently understood and easily carried out by those skilled in the art in the technical field of the present application or in a similar technical field.

### Example 1: Construction of random mutation library using transposon

### Example 1-1: Construction of Corynebacterium glutamicum strain having L-glutamic acid producing ability derived from wild-type Corynebacterium glutamicum

In order to produce a strain having an L-glutamic acid producing ability derived from the wild-type *Corynebacterium glutamicum* ATCC13869, a *Corynebacterium glutamicum* ATCC13869ΔodhA strain, in which the *odhA* gene was deleted, was prepared based on the prior literature (Appl Environ Microbiol. 2007 Feb;73(4): 1308-19. Epub 2006 Dec 8.).

Specifically, in order to delete the *odhA* gene, the upstream and downstream regions of the *odhA* gene were obtained through PCR using the primer sets of SEQ ID NO: 3 and SEQ ID NO: 4, and SEQ ID NO: 5 and SEQ ID NO: 6 based on the wild-type *Corynebacterium glutamicum* ATCC13869 chromosomal DNA as a template. Solg^{™} Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 55°C for 30 seconds, and polymerization at 72°C for 60 seconds, and then polymerization at 72°C for 5 minutes.

The upstream and downstream regions of the amplified *odhA,* and the pDCM2 vector for chromosomal transformation cleaved by Smal restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant vector, and the resulting vector was named pDCM2-ΔodhA. The cloning was performed by mixing the Gibson assembly reagent and each of the gene fragments in a calculated number of moles, followed by incubating at 50°C for 1 hour.

The thus-prepared pDCM2-ΔodhA vector was transformed into the *Corynebacterium glutamicum* ATCC13869 strain by electroporation and subjected to secondary crossover to thereby obtain a strain in which the *odhA* gene was deleted on the chromosome. The deletion of the gene was confirmed through genome sequencing and PCR using SEQ ID NO: 7 and SEQ ID NO: 8, and the resulting strain was named ATCC13869ΔodhA.

The primer sequences used herein are shown in Table 1 below.

**[Table 1]**

| SEQ ID NO: | Name | Sequence |
|---|---|---|
| 3 | odhA_up_F | TGAATTCGAGCTCGGTACCCTTGAACGGAATTGGGTGG |
| 4 | odhA_up_R | CCCAGGTGGCATCGGTACCTTCACCCAGCGCCACGCAG |
| 5 | odhA_down_F | CGCTGGGTGAAGGTACCGATGCCACCTGGGTTGGTCAAG |
| 6 | odhA_down_R | GTCGACTCTAGAGGATCCCCGGACAAGGAATGGAGAGA |
| 7 | odhA_del_F | CTTACCGTTGTTGCCCTT |
| 8 | odhA_del_R | CTCCTTCACCCACATCATT |

### Example 1-2: Construction of random mutation library using transposon

In order to obtain a strain having an increased L-glutamic acid producing ability, a vector library was constructed in the following manner.

The plasmids obtained using EZ-Tn5^{™} <R6Kγori/KAN-2>Tnp Transposome^{™} Kit (Epicentre) were transformed based on the *Corynebacterium glutamicum* ATCC13869ΔodhA as the parent strain by electroporation(Appl. Microbiol. Biothcenol.(1999) 52:541-545) and spread on a complex medium plate containing kanamycin (25 mg/L) to thereby obtain about 5,000 colonies.

### <Complex Medium Plate (pH 7.0)>

Glucose 10 g, Peptone 10 g, Beef extract 5 g, Yeast extract 5 g, Brain Heart Infusion 18.5 g, NaCl 2.5 g, Urea 2 g, Sorbitol 91 g, Agar 20 g (per liter of distilled water)

### Example 2: Random Mutation Library Screening Using Transposon

Each of about 5,000 colonies obtained in Example 1-2 was inoculated onto 300 µL of the following selective medium and cultured in a 96-deep-well plate at 37°C at 1000 rpm for about 48 hours.

### < Selective Medium (pH 7.0)>

Raw sugar 5%, 1 M Phosphate buffer (pH 8.0) 100 mL, HSM (hydrolyzed soybean meal) 0.096%, Ammonium sulfate 2.25%, Monopotassium phosphate 0.1%, Magnesium sulfate 0.04%, Iron sulfate 10 mg/L, Thiamine-HCL 0.2 mg/L, Biotin 0.3 mg/L (per liter of distilled water)

After completion of the culture, L-glutamic acid was measured through YSI (YSI 2900 Biochemistry Analyzer). 10 colonies were selected as mutant strains showing a high L-glutamic acid value as compared to the *Corynebacterium glutamicum* ATCC13869ΔodhA, the parent strain. Other colonies showed an L-glutamic acid value similar to or lower than that of the *Corynebacterium glutamicum* ATCC13869ΔodhA strain used as the parent strain.

The selected 10 strains were cultured again in the same manner as above, and as a result, the top one mutant strain ATCC13869ΔodhA/mt-8 with an enhanced L-glutamic acid producing ability as compared to the *Corynebacterium glutamicum* ATCC13869ΔodhA, the parent strain, was selected.

### Example 3: Identification of causes of increase in L-glutamic acid producing ability of selected mutant strain

Based on the ATCC13869ΔodhA/mt-8 selected in Example 2, the gene deleted by random insertion of the transposon was analyzed using the primer 1 (SEQ ID NO: 9) and primer 2 (SEQ ID NO: 10) of the manufacturer's manual and kit. As a result of the analysis, it was confirmed that the gene (*BBD29_04485*) including the polynucleotide sequence of SEQ ID NO: 2 was inactivated based on the nucleotide sequences registered in the NIH GenBank.

The primer sequences used herein are shown in Table 2 below.

**[Table 2]**

| SEQ ID NO: | Name | Sequence |
|---|---|---|
| 9 | Primer 1 | ACCTACAACAAAGCTCTCATCAACC |
| 10 | Primer 2 | CTACCCTGTGGAACACCTACATCT |

### Example 4: Construction of recombinant vector for VKOR protein inactivation

### Example 4-1: Construction of recombinant vector for deletion of BBD29_04485 gene encoding VKOR protein

As confirmed in Example 3, a gene-deleted recombinant vector was prepared to confirm whether the L-glutamic acid producing ability was enhanced when the *BBD29_04485* gene (SEQ ID NO: 2) encoding the VKOR protein was deleted on the chromosome of the strain of the genus *Corynebacterium.*

To this end, primers of SEQ ID NOS: 11 to 14 were first synthesized to prepare a fragment for the deletion of the gene.

Specifically, *BBD29_04485* gene fragments were obtained through PCR using the primer sets of SEQ ID NO: 11 and SEQ ID NO: 12, and SEQ ID NO: 13 and SEQ ID NO: 14 based on the wild-type *Corynebacterium glutamicum* ATCC13869 chromosomal DNA as a template. Solg^{™} Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 55°C for 30 seconds, and polymerization at 72°C for 60 seconds, and then polymerization at 72°C for 5 minutes.

The amplified gene fragments and the pDCM2 vector for chromosomal transformation cleaved by Smal restriction enzyme were cloned using the Gibson assembly method to obtain a recombinant vector, and the resulting vector was named pDCM2-ΔBBD29_04485. The cloning was performed by mixing the Gibson assembly reagent and each of the gene fragments in a calculated number of moles, followed by incubating at 50°C for 1 hour.

The primer sequences used herein are shown in Table 3 below.

**[Table 3]**

| SEQ ID NO: | Name | Sequence |
|---|---|---|
| 11 | BBD29_044 85_up_F | AATTCGAGCTCGGTACCCGTGAGTCCAACCAGGTCGAAG |
| 12 | BBD29_044 85_up_R | CTGAAAGCCTTAAATGCTGGCTTGATTTCTTGTGCTGTG |
| 13 | BBD29_044 85_down_F | ACAAGAAATCAAGCCAGCATTTAAGGCTTTCAGGCCG |
| 14 | BBD29_044 85_down_R | CGACTCTAGAGGATCCCCGTATCGACAAGGGTGAGTGATG |

### Example 4-2: Construction of recombinant vectors for changing initiation codon of BBD29_04485 encoding VKOR protein

In order to confirm whether the L-glutamic acid producing ability was enhanced when the initiation codon of the *BBD29_04485* gene was changed to each of TTG and CTG on the chromosome of the strain of the genus *Corynebacterium,* recombinant vectors for changing the initiation codon was first prepared.

To this end, primers of SEQ ID NOS: 15 to 20 were synthesized in order to prepare a fragment for changing the initiation codon of the gene.

Specifically, gene fragments were obtained through PCR using the primer sets of SEQ ID NO: 15 and SEQ ID NO: 16, SEQ ID NO: 17 and SEQ ID NO: 18, SEQ ID NO: 15 and SEQ ID NO: 19, and SEQ ID NO: 18 and SEQ ID NO: 20 based on the wild-type *Corynebacterium glutamicum* ATCC13869 chromosomal DNA as a template. Solg^{™} Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 55°C for 30 seconds, and polymerization at 72°C for 60 seconds, and then polymerization at 72°C for 5 minutes.

The amplified gene fragments and the pDCM2 vector for chromosomal transformation cleaved by Smal restriction enzyme were cloned using the Gibson assembly method to obtain recombinant vectors, and the resulting vectors were named pDCM2-ABBD29_04485::BBD29_04485(g1t) vector and pDCM2-ABBD29_04485::BBD29_04485(g1c) vector. The cloning was performed by mixing the Gibson assembly reagent and each of the gene fragments in a calculated number of moles, followed by incubating at 50°C for 1 hour.

The primer sequences used herein are shown in Table 4 below.

**[Table 4]**

| SEQ ID NO: | Name | Sequence |
|---|---|---|
| 15 | BBD29_044 85_up_F_2 | TTCGAGCTCGGTACCCGTTGATGATCAGGGAAGGCTGT |
| 16 | BBD29_044 85_up_R_2 | TGGATTTCGGTAGACAaGGGGAGACCTCCGGGTGGGAA |
| 17 | BBD29_044 85_down_F _2 | ACCCGGAGGTCTCCCCtTGTCTACCGAAATCCACAACG |
| 18 | BBD29_044 85_down_R _2 | ACTCTAGAGGATCCCCTCGGTCAGGTGTGTGTAAATAG |
| 19 | BBD29_044 85_up_R_3 | TGGATTTCGGTAGACAGGGGGAGACCTCCGGGTGGGAA |
| 20 | BBD29_044 85_down_F _3 | ACCCGGAGGTCTCCCCCTGTCTACCGAAATCCACAACG |

### Example 4-3: Construction of recombinant vectors for changing ribosome binding site (RBS) of BBD29_04485 encoding VKOR protein

In order to weaken the *BBD29_04485* gene on the chromosome of the strain of the genus *Corynebacterium,* Shine-Dalgarno sequence (SD) was predicted from the nucleotide sequences containing the top 35 base pairs of the gene and 35 base pairs from the N-terminus of the open reading frame (ORF).

Based on the above nucleotide sequences, a total of three ribosome binding sequence candidate groups, namely, RBS1, RBS2, and RBS3, were predicted through the RBS calculator (github). As a result, it was predicted that the expression of the three ribosome binding sequence candidate groups was reduced by 50%, 20%, and 10%, respectively, compared to the existing ribosome binding sequences.

The predicted ribosome binding sequences are shown in Table 5.

**[Table 5]**

| Name | Shine-Dalgarno Sequence | Predicted Expression Level | Expression Level (%) |
|---|---|---|---|
| Original | ACCCGGAGG | 13122 | 100% |
| RBS1 | CTCGAAAGT | 6581 | 50% |
| RBS2 | ACTCGAGGC | 2620 | 20% |
| RBS3 | GCCCTGGAC | 1312 | 10% |

In order to confirm whether the L-glutamic acid producing ability was enhanced when the gene was weakened by changing the ribosome binding sequence of *BBD29_04485* on the chromosome of the strain of the genus *Corynebacterium,* recombinant vectors for changing the ribosome binding sequences for each of the three predicted ribosome binding sequences were prepared.

To this end, primers of SEQ ID NOS: 21 to 28 were first synthesized in order to prepare a fragment for changing the ribosome binding sequences of the gene.

Specifically, gene fragments were obtained through PCR using the primer sets of SEQ ID NO: 21 and SEQ ID NO: 22, SEQ ID NO: 23 and SEQ ID NO: 24, SEQ ID NO: 21 and SEQ ID NO: 25, SEQ ID NO: 24 and SEQ ID NO: 26, SEQ ID NO: 21 and SEQ ID NO: 27, and SEQ ID NO: 24 and SEQ ID NO: 28 based on the wild-type *Corynebacterium glutamicum* ATCC13869 chromosomal DNA as a template. Solg^{™} Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 55°C for 30 seconds, and polymerization at 72°C for 60 seconds, and then polymerization at 72°C for 5 minutes.

The amplified gene fragments and the pDCM2 vector for chromosomal transformation cleaved by Smal restriction enzyme were cloned using the Gibson assembly method to obtain recombinant vectors, and the resulting vectors were named pDCM2-ΔRBS(wt)::RBS1 vector, pDCM2-ΔRBS(wt)::RBS2 vector, and pDCM2-ΔRBS(wt)::RBS3 vector. The cloning was performed by mixing the Gibson assembly reagent and each of the gene fragments in a calculated number of moles, followed by incubating at 50°C for 1 hour.

The primer sequences used herein are shown in Table 6 below.

**[Table 6]**

| SEQ ID NO: | Name | Sequence |
|---|---|---|
| 21 | BBD29_04485 _up_F_4 | TTCGAGCTCGGTACCCCAGCATCTTCTTCTTCGCCGAA |
| 22 | BBD29_04485 _up_R_4 | GTAGACACGGGGAGAACTTTCGAGGGGAAAACGTTTGTTG |
| 23 | BBD29_04485 _down_F_4 | AACAAACGTTTTCCCCTCGAAAGTTCTCCCCGTGTCTACC |
| 24 | BBD29_04485 _down_R_4 | ACTCTAGAGGATCCCCCTTTTCTGAGCTGGAGGAACAA |
| 25 | BBD29_04485 _up_R_5 | GTAGACACGGGGAGAGCCTCGAGTGGGAAAACGTTTGTTG |
| 26 | BBD29_04485 _down_F_5 | AACAAACGTTTTCCCACTCGAGGCTCTCCCCGTGTCTACC |
| 27 | BBD29_04485 _up_R_6 | GTAGACACGGGGAGAGTCCAGGGCGGGAAAACGTTTGTTG |
| 28 | BBD29_04485 _down_F_6 | AACAAACGTTTTCCCGCCCTGGACTCTCCCCGTGTCTACC |

### Example 5: Construction of strain having L-glutamic acid producing ability derived from wild-type Corynebacterium glutamicum in which BBD29 04485 encoding VKOR protein is weakened

The effect on the L-glutamic acid producing ability was confirmed by introducing the pDCM2-ΔBBD29_04485 vector, pDCM2-ΔBBD29_04485::BBD29_04485(g1t) vector, pDCM2-ABBD29_04485::BBD29_04485(g1c) vector, pDCM2-ΔRBS(wt)::RBS1 vector, pDCM2-ΔRBS(wt)::RBS2 vector, and pDCM2-ΔRBS(wt)::RBS3 vector prepared in Example 4 based on the ATCC13869ΔodhA strain prepared in Example 1.

First, the pDCM2-ΔBBD29_04485 vector was transformed into the *Corynebacterium glutamicum* ATCC13869ΔodhA strain by electroporation and subjected to secondary crossover to thereby obtain a strain in which the *BBD29_04485* gene was deleted on the chromosome. The gene manipulation was confirmed through genome sequencing and PCR using SEQ ID NO: 29 and SEQ ID NO: 30, which can amplify the homologous recombinant upstream and downstream regions, respectively, and the resulting strain was named CA02-1624.

Next, the pDCM2-ΔBBD29_04485::BBD29_04485(g1t) vector and pDCM2-ΔBBD29_04485::BBD29_04485(g1c) vector were transformed into the *Corynebacterium glutamicum* ATCC13869ΔodhA strain by electroporation and subjected to secondary crossover to thereby obtain strains in which the initiation codon of the *BBD29_04485* gene was changed from GTG to TTG and CTG, respectively.

The gene manipulation was confirmed through genome sequencing and PCR using SEQ ID NO: 31 and SEQ ID NO: 30, which can amplify the homologous recombinant upstream and downstream regions, respectively, and the resulting strains were named CA02-1625 and CA02-1630.

Next, the pDCM2-ΔRBS(wt)::RBS1 vector, pDCM2-ΔRBS(wt)::RBS2 vector, and pDCM2-ΔRBS(wt)::RBS3 vector were transformed into the *Corynebacterium glutamicum* ATCC13869ΔodhA strain by electroporation and subjected to secondary crossover to thereby obtain strains in which the ribosome binding sequence of the *BBD29_04485* gene was changed to RBS1, RBS2, and RBS3 on the chromosome, respectively.

The gene manipulation was confirmed through genome sequencing and PCR using SEQ ID NO: 31 and SEQ ID NO: 30, which can amplify the homologous recombinant upstream and downstream regions, respectively, and the resulting strains were named CA02-1631, CA02-1632, and CA02-1633.

The primer sequences used herein are shown in Table 7.

**[Table 7]**

| SEQ ID NO: | Name | Sequence |
|---|---|---|
| 29 | BBD29_04485_del_ F | ATAATCCACCAAAACTGCC |
| 30 | BBD29_04485_del_ R | AGGTGTGTGTAAATAGGGA |
| 31 | BBD29_04485_seq _F | GTGGTTGTCTTCGATAGT |

A total of 6 strains, CA02-1624, CA02-1625, CA02-1630, CA02-1631, CA02-1632, and CA02-1633, were cultured in the following manner in order to confirm the L-glutamic acid producing ability, based on the ATCC13869ΔodhA strain as a control.

The above strains were inoculated on a complex medium plate consisting of a seed medium and cultured at 30°C for 20 hours. Then, the above strains was inoculated into a 250 mL corner-baffled flask containing 25 mL of the production medium below using an inoculation loop, and then cultured with shaking at 200 rpm at 30°C for 40 hours. After completion of the culture, L-glutamic acid production was measured by high-performance liquid chromatography (HPLC), and the measurement results are shown in Table 8 below.

### <Seed Medium>

Glucose 1%, Beef extract 0.5%, Polypeptone 1%, Sodium chloride 0.25%, Yeast extract 0.5%, Agar 2%, Urea 0.2%, pH 7.2

### <Production Medium>

Raw sugar 6%, Calcium carbonate 5%, Ammonium sulfate 2.25%, Monopotassium phosphate 0.1%, Magnesium sulfate 0.04%, Iron sulfate 10mg/L, Thiamine-HCL 0.2 mg/L, Biotin 50 µg/L

**[Table 8]**

| Name of Strains | L-Glutamic Acid Concentration (g/L) | L-Glutamic Acid Concentration Increase Rate (%) |
|---|---|---|
| ATCC13869ΔodhA | 2.1 | - |
| CA02-1624 | 3.2 | 52.4% |
| CA02-1625 | 2.7 | 28.6% |
| CA02-1630 | 3.0 | 42.9% |
| CA02-1631 | 2.4 | 14.3% |
| CA02-1632 | 2.8 | 33.3% |
| CA02-1633 | 3.0 | 42.9% |

As shown in Table 8, it was confirmed that the concentration of L-glutamic acid was increased in all of the CA02-1624 strain, in which the *BBD29_04485* gene was deleted, the CA02-1625 and CA02-1630 strains, in which the initiation codon of the *BBD29_04485* gene was weakened to TTG and CTG, and the CA02-1631, CA02-1632, and CA02-1633 strains, in which the ribosome binding sequence of the *BBD29_04485* gene was weakened to RBS1, RBS2, and RBS3, as compared to the wild-type-derived ATCC13869ΔodhA strain.

Based on the results, it can be seen that the L-glutamic acid producing ability is enhanced as long as the activity of the VKOR protein is inactivated regardless of the specific means.

Among the strains, CA02-1624 was deposited at the Korean Culture Center of Microorganisms under Budapest Treaty on January 13, 2021 with Accession No. KCCM12928P.

### Example 6: Confirmation of effect of deleting BBD29 04485 encoding VKOR protein in N-methyl-N'-nitro-N-nitrosoguanidine (NTG)-mutant Corynebacterium glutamicum strain

In addition to the strains derived from the wild-type *Corynebacterium* sp., in order to confirm whether the gene exhibits the same effect in the NTG-mutant strain derived from *Corynebacterium* sp. with an increased L-glutamic acid producing ability, the attenuation effect of the gene confirmed in Example 5 was confirmed in the *Corynebacterium glutamicum* BL2 strain known as the L-glutamic acid-producing NTG mutant strain (KFCC1 1074, Korean Patent No. 10-0292299).

Specifically, the effect on L-glutamic acid producing ability was confirmed by introducing a total of 3 vectors, the pDCM2-ΔBBD29_04485 vector, the pDCM2-ABBD29_04485::BBD29_04485(g1c) vector, and the pDCM2-ΔRBS(wt)::RBS3 vector showing an increased L-glutamic acid concentration by 40% or more in Example 5, into the KFCC1 1074 strain.

First, the pDCM2-ΔBBD29_04485 vector was transformed into the KFCC11074 strain by electroporation and subjected to secondary crossover to thereby obtain a strain in which the *BBD29_04485* gene was deleted on the chromosome.

The gene manipulation was confirmed through genome sequencing and PCR using SEQ ID NO: 29 and SEQ ID NO: 30, which can amplify the homologous recombinant upstream and downstream regions, respectively, and the resulting strain was named CA02-1634.

Next, the pDCM2-ABBD29_04485::BBD29_04485(g1c) vector was transformed into the KFCC1 1074 strain by electroporation and subjected to secondary crossover to thereby obtain a strain in which the initiation codon of the *BBD29_04485* gene was changed from GTG to CTG.

The gene manipulation was confirmed through genome sequencing and PCR using SEQ ID NO: 31 and SEQ ID NO: 30, which can amplify the homologous recombinant upstream and downstream regions, respectively, and the resulting strain was named CA02-1635.

Next, the pDCM2-ΔRBS(wt)::RBS3 vector was transformed into the KFCC1 1074 strain by electroporation and subjected to secondary crossover to thereby obtain a strain in which the ribosome binding sequence of the *BBD29_04485* gene was changed to RBS3 on the chromosome.

The gene manipulation was confirmed through genome sequencing and PCR using SEQ ID NO: 31 and SEQ ID NO: 30, which can amplify the homologous recombinant upstream and downstream regions, respectively, and the resulting strain was named CA02-1636.

A total of 3 prepared strains, CA02-1634, CA02-1635 and CA02-1636, were cultured in the following manner in order to confirm the L-glutamic acid producing ability, based on the KFCC1 1074 strain as a control.

The above strains were inoculated on a complex medium plate consisting of a seed medium and cultured at 30°C for 20 hours. Then, the above strains was inoculated into a 250 mL corner-baffled flask containing 25 mL of the production medium below using an inoculation loop, and then cultured with shaking at 200 rpm at 30°C for 40 hours. After completion of the culture, L-glutamic acid production was measured by high-performance liquid chromatography (HPLC), and the measurement results are shown in Table 9 below.

### <Seed Medium>

Glucose 1%, Beef extract 0.5%, Polypeptone 1%, Sodium chloride 0.25%, Yeast extract 0.5%, Agar 2%, Urea 0.2%, pH 7.2

### <Production Medium>

Raw sugar 6%, Calcium carbonate 5%, Ammonium sulfate 2.25%, Monopotassium phosphate 0.1%, Magnesium sulfate 0.04%, Iron sulfate 10mg/L, Thiamine-HCL 0.2 mg/L, Biotin 500 µg/L

**[Table 9]**

| Name of Strains | L-Glutamic Acid Concentration (g/L) | L-Glutamic Acid Concentration Increase Rate (%) |
|---|---|---|
| KFCC11074 | 6.9 | - |
| CA02-1634 | 10.1 | 46.4% |
| CA02-1635 | 9.5 | 37.7% |
| CA02-1636 | 9.1 | 31.9% |

As shown in Table above, it was confirmed that the concentration of L-glutamic acid was increased by about 46.4% in CA02-1634, in which the *BBD29_04485* gene was deleted, compared to the KFCC1 1074 strain. In addition, it was confirmed that the concentration of L-glutamic acid was increased by about 37.7% in CA02-1635, in which the initiation codon of the *BBD29_04485* gene was weakened to CTG. Further, it was confirmed that the concentration of L-glutamic acid was increased by about 31.9% in CA02-1636, in which the ribosome binding sequence of the *BBD29_04485* gene was weakened to RBS3.

Those of ordinary skill in the art to which the present application belongs will recognize that the present application may be embodied in other specific forms without departing from its spirit or essential characteristics. The described embodiments are to be considered in all respects only as illustrative and not restrictive. The scope of the present application is therefore indicated by the appended claims rather than by the foregoing description. All changes which come within the meaning and range of equivalency of the claims are to be embraced within the scope of the present application.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA02-1624 | Accession number given by the
INTERNATIONAL DEPOSITARY AUTHORITY:
KCCM12928P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 13. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 13. 2021.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A microorganism of the genus *Corynebacterium,* in which VKOR protein (vitamin K epoxide reductase family protein) is inactivated.

2. The microorganism of claim 1, wherein the VKOR protein is derived from the genus *Corynebacterium.*

3. The microorganism of claim 1, wherein the VKOR protein consists of a polypeptide represented by an amino acid sequence having an identity of 80% or more with an amino acid sequence of SEQ ID NO: 1.

4. The microorganism of claim 1, wherein the VKOR protein is encoded by a polynucleotide represented by a nucleotide sequence of SEQ ID NO: 2.

5. The microorganism of claim 1, wherein the inactivation of VKOR protein means deletion or weakening of the polynucleotide represented by the nucleotide sequence of SEQ ID NO: 2.

6. The microorganism of claim 1, wherein the microorganism of the genus *Corynebacterium* is *Corynebacterium glutamicum.*

7. The microorganism of claim 1, wherein the microorganism has an increased L-glutamic acid producing ability compared to a parent strain or wild-type strain in which the VKOR protein is not inactivated.

8. The microorganism of claim 1, wherein the microorganism comprises OdhA protein which is further inactivated.

9. A method for producing L-glutamic acid, comprising: culturing a microorganism of the genus *Corynebacterium,* in which VKOR protein is inactivated, in a medium.

10. The method of claim 9, wherein the VKOR protein is derived from the genus *Corynebacterium.*

11. The method of claim 9, wherein the VKOR protein consists of a polypeptide represented by an amino acid sequence having an identity of 80% or more with an amino acid sequence of SEQ ID NO: 1.

12. The method of claim 9, wherein the VKOR protein is encoded by a polynucleotide represented by a nucleotide sequence of SEQ ID NO: 2.

13. The method of claim 9, wherein the inactivation of VKOR protein means deletion or weakening of the polynucleotide represented by the nucleotide sequence of SEQ ID NO: 2.

14. The method of claim 9, wherein the microorganism of the genus *Corynebacterium* is *Corynebacterium glutamicum.*

15. The method of claim 9, wherein the microorganism comprises OdhA protein which is further inactivated.
